# EUROPEAN PATENT SPECIFICATION

(11) **EP 4 456 857 B1**
(45) Date of publication and mention of the grant of the patent: **20.08.2025**
(21) Application number: 22835492.4
(22) Date of filing: 21.12.2022
(51) Int. Cl.: A61H 15/00, A61H 7/00

(54) **MAGNETOTHERAPY AND COMPRESSIVE MICROVIBRATION APPARATUS FOR THE TISSUES**
MAGNETOTHERAPIE- UND KOMPRESSIVE MIKROVIBRATIONSVORRICHTUNG FÜR GEWEBE
APPAREIL DE MAGNÉTOTHÉRAPIE ET DE MICROVIBRATION COMPRESSIVE POUR LES TISSUS

(30) Priority: 28.12.2021 IT 202100032744
(43) Date of publication of application: 06.11.2024
(73) Proprietor: Fenix Group S.r.l., 65015 Montesilvano (PE) (IT)
(72) Inventor: CAVALLETTI, Gianluca, 65015 Montesilvano (PE) (IT)
(74) Representative: Fiammenghi, Eva
(86) International application number: PCT/IB2022/062577
(87) International publication number: WO 2023/126775

(56) References cited:
- WO-A1-2019/168444
- WO-A1-2020/148624
- US-A1- 2020 054 890
- US-A1- 2021 146 119

## Description

### Technical field

The present patent application for an invention generally relates to the field of aesthetic medicine, sports medicine and phlebo-lymphology.

The invention is applicable to any field where such a type of device can be advantageously used, but preferably it relates to the field of the treatment of the various forms of cellulite and lipolyniphedenia.

### Prior art

As is known, cellulite manifests itself from an aesthetic point of view in the form of irregularities on the surface of the epidermis and is scientifically referred to as edematous-fibrosclerotic panniculopathy.

Cellulite indicates an alteration of the subcutaneous tissue rich in fat cells and is characterized precisely by the increase in the volume of fat cells, where excess liquids accumulate in the intracellular spaces, which are left over from the body's biochemical processes. The balance of the venous and lymphatic systems is modified by a slowing down of blood flow and fluid retention by the tissues.

The onset of cellulite, even if basically everything can be traced back to an alteration of the microcirculation, can be considered dependent on various factors which often add up to each other. Some of these factors cannot be eliminated and are therefore defined as primary as they are due to sex, race or familiarity, others are defined as secondary as they are connected to certain phases of life, particular pathologies or the intake of drugs and others still aggravating factors are defined as well as poor diet or sedentary lifestyle and which could certainly be controlled by adopting a different lifestyle. Since the 1970s, cellulite is no longer classified as a generic imperfection, but as an autonomous pathological condition.

Treatment of cellulite includes surgical methods and non-invasive methods.

The operation to surgically eliminate cellulite is generically called liposuction and involves the effective removal of fat deposits in limited and fairly extensive body areas, also with the use of general anesthesia to reach the deeper layers. Obviously it is a technique, which although valid, is very invasive and therefore indicated only in the most serious cases.

Leaving aside the treatments with anti-cellulite creams, which are not directly part of the present invention, there are many non-invasive treatments to cure cellulite and which consist of medical techniques that use equipment such as cavitation, ultrasound, radiofrequency, electrolipolysis, cryosculpture, laser therapy, ozone therapy, pressotherapy, lymphatic drainage and magnetotherapy. These techniques are widely practiced both in the professional field and directly by the patient and are more or less effective in resolving or at least reducing this imperfection.

A magnetotherapy and vibration apparatus is disclosed in US 2020/054890 A1.

Starting from the consideration that for years the technique of dermo-suction has been used, thanks to which the tissues were sucked up and placed in traction by means of cylinders, this technology has gradually evolved using less bloody techniques for the purpose which, both in the professional field and directly by the user, were based on the well-known compressive micro-vibration technology of the tissues, but which resulted to be variously effective in solving or at least reducing the type of imperfection reported above.

Therefore, the main object of the present invention consists in general in realizing a device based on the well-known technology of compressive micro-vibration of the tissues, which is able to act even more effectively on the reduction of this imperfection, compared to those existing in the prior art and that adopt this technology.

A further object of the present invention is to provide to all those who carry out a professional activity, relating to the treatment of this type of blemish, a device which is capable, with the same apparatus, of implementing in synergy at least two of the techniques previously mentioned, in order to be able to effectively extend the treatment to one type of disorder or synergistically to several types of disorders.

Therefore, the object of the present invention consists in general in realizing a device designed to solve the above problems, as will result from the detailed description of an exemplifying and non-limiting embodiment thereof, illustrated below.

### Summary of the invention

The present patent application for industrial invention is intended to describe and claim an apparatus provided with at least a new and alternative solution to the solutions known to date and in particular it aims to obviate one or more of the drawbacks or problems referred to above and/or to meet one or more needs perceived in the art and in particular deducible from the above.

For this purpose, the inventors have developed an effective medical device, capable of realizing the benefits deriving from a combined magnetotherapy treatment and an improved compressive microvibration treatment of the tissues.

Specifically, said medical device can be advantageously used to improve disorders associated with vascular hemodynamics, increase in interstitial fibrous tissue, lymphatic stasis and lymphedema, and painful conditions deriving from edematous conditions.

In particular, the well-known technology of compressive microvibration of the tissues implemented by means of a handpiece, inside which a rotor consisting of multiple silicone balls is housed, is effectively and inventively implemented and perfected using, as described in detail below, multiple balls having a particular internal and external conformation associated with calibrated elastomeric features, capable of transferring pressure on the body area being treated in a controlled manner, by virtue of their particular viscoelastic nature. Specifically, a particularly fundamental feature, according to the present invention, relates to the fact that said calibrated more or less elastomeric features of the balls or better of each row containing balls having the same viscoelastic properties represents a means by which to obtain compressive harmonic waves determined on the body area being treated in addition to the classic compression effect already existing in the art. Even more specifically, said compressive microvibration technology of the tissues modified by the formation of compressive harmonic waves on the body area being treated is due to a hypercompressive effect, which exploits the muscle as active resistance when said body tissue comes into contact cyclically with the balls having high rigidity during the rotation of the rotor. Therefore, it is intuitive to consider that the frequency, duration and amplitude of said compressive harmonic waves derive directly from the arrangement of said row of balls having high rigidity and arranged between the rows having balls with viscoelastic features.

Another innovative feature consists in that the benefits deriving from the aforementioned modified technology of compressive microvibration of the tissues are effectively implemented by combining it with the physical therapy of magnetotherapy and all this by designing said handpiece in order to accommodate a specially conceived solenoid capable of emitting a suitable electromagnetic field to implement a magnetotherapy treatment in synergy.

Although the benefits deriving from the compressive microvibration treatment of the tissues consisting of a lymphatic drainage action, accompanied by a remodeling action, belong to the prior art, by virtue of the vibration and compression exerted by the aforementioned elastomeric balls, it is also appropriate to briefly refer to the benefits brought about by a combined magnetic therapy treatment. Magnetotherapy seems to be able to stimulate tissue regeneration, due to the restoration of a cellular biochemical balance exclusively and effectively for therapeutic and curative purposes. For example, it seems to be effective as an adjuvant treatment against cellulite, precisely because it acts directly on the adipocytes, allowing a strong metabolic stress and locally on the blood circulation to be induced, reducing water retention and inflammation that accompany cellulite itself.

The present invention therefore achieves its main objects which consist in a greater effectiveness of action, realizing a handpiece capable of realizing the benefits deriving from a combined magnetotherapy treatment and a modified compressive microvibration treatment of the tissues, using a single handpiece and all governed by electronic and IT systems capable of ensuring a high level of effectiveness, safety, precision and correctness of the treatment.

The present invention therefore relates to a magnetotherapy and compressive microvibration apparatus for the tissues comprising a handpiece consisting of a head, a body and a handle, which is suitable for housing in its recess a specially designed rotor having an arrangement which permits it to carry out work in a radial direction with respect to the axis of the access hole.

Said rotor is shaped like a hollow cylinder on the lateral surface of which elastomeric rotating balls are placed which rotate on themselves through a passive movement induced by sliding on the tissue, and through an active mechanical rotary movement induced by the roller and managed by the handpiece. Said elastomeric balls are supported on pins common to at least four balls and whose rotation axis coincides with that of insertion into the rotor and in which said pins are constrained respectively to a head ring nut and to a ring nut of the handle. Said ring nut of the handle comprises a fitting system adapted to be inserted on a rotation mechanism forming part of an electrically operated motor and housed within said handle of the handpiece and connected by means of a cable to a computer terminal.

As already stated, the rotation of the rotor is such as to cause the passive rotation of each individual ball on itself, each time it comes into contact with the patient's integumentary apparatus, through a rectangular opening made on said body of the handpiece. Specifically, a fundamental feature of the invention concerns the fact that the system is suitable for ensuring that by placing the handpiece on the dermis only with its weight, the mechanical resistance encountered by the rotor balls while sliding on the patient's tissue is detected by means of a dedicated system of pressure and speed sensors, thus detecting the type of edematous or fibrous tissue with which the balls are in contact and thus independently regulating the intensity of the device according to the resistance. In other words, said device inventively presents a mechanism for adjusting the intensity of the device according to the resistance encountered on the tissue and therefore relating to the presence of more or less incompressible fibrous areas and which represent an unequivocal sign of cellulite areas.

The particular honeycomb arrangement and rather the particular internal and external conformation of said elastomeric balls conferring specific viscoelastic capacities, as well as the presence of a row with rigid balls, also allows, during the rotation implemented by the rotor and during the passage of the handpiece on the tissue of a patient, obtaining both a tissue compressive action and a vibration action consisting of a series of rapid movements transmitted to the tissues themselves with a succession of hypercompressions and lifting which create a succession of amplified compressive waves and ensuring that the contact between the balls and the skin tissue is never interrupted.

Another innovative concept underlying the present invention provides for the presence, at the area of the body of the handpiece between the handle and the rectangular opening, of a temperature sensor capable of detecting the temperature of the patient's integumentary system and therefore detect areas of greater or lesser blood perfusion.

An interface comprising a display, a plurality of indicators and buttons which allows the operator to interact with a dedicated software is made on the body of the handpiece in a position opposite to that of the rectangular opening. A preferred embodiment of the invention provides that said interface consists of a single touch screen, which allows the operator to interact with a graphical interface, capable of realizing the manual display and control of all the above parameters.

In addition to being an indispensable and simple means of displaying the parameters in real time, this interface also allows the operator to be able to instantly vary all those parameters set at the start of the session using the dedicated software, using a standard basic setting or better data present in the memory and obtained from the previous sessions on that patient, as well as the real-time speed of the rotor, the intensity of the impulses of the inductor and the feedback mechanism, which links the automatic variation of the speed of the rotor on the basis of temperature and resistance values that the balls encounter during contact with the tissue or with more or less fibrotic areas being treated.

The set of programs used in the data processing system and which creates the dedicated software includes a program capable of analyzing the data detected by the temperature sensor already after the first treatment and of drawing up a thermal map of the patient's body areas, which will be viewed by the operator before each treatment session to understand both on which areas the treatment should be concentrated more on the basis of the pathology to be treated and to configure the dedicated software with the necessary range of values.

It is therefore a specific object of the present invention to develop an effective medical apparatus including the relative dedicated software, capable of realizing the benefits deriving from a combined magnetotherapy treatment and a compressive microvibration treatment of the tissues in the treatment or alleviation of aesthetic disorders resulting from cellulite.

A further and more general object is to be able to realize a combined magnetotherapy and compressive microvibration treatment of the tissues capable of being advantageously used in the improvement of disorders associated with vascular hemodynamics, the increase in interstitial fibrous tissue, lymphatic stasis and lymphedema, to painful states resulting from edematous conditions.

Other features of the present invention are described in the following detailed description of one or more specific embodiments thereof, protected by the various dependent claims.

### Brief description of the drawings

The foregoing advantages, as well as other advantages and features of the present invention, will be illustrated with reference to the accompanying drawings, which are to be considered purely illustrative and not limiting or binding to the effects of the present patent application, in which:
- FIGURE 1A is a front view of the handpiece forming part of the inventive magnetotherapy and compressive microvibration apparatus for the tissues,
- FIGURE 1B is a front sectional view of the handpiece of Fig. 1;
- FIGURE 2 is an exploded view of the handpiece of Fig. 1;
- FIGURE 3 shows in detail the exploded view of the rotor installed in the handpiece according to the invention;
- FIGURE 4 shows in detail the exploded view of the inductor and the plug of the handpiece according to the invention;
- FIGURE 5 shows in detail the exploded view of the interface and of the isolating and clamping support means of the handpiece cable according to the invention;
- FIGURE 6 shows in detail in A the perspective view of an elastomeric ball and in B the plan view of the elastomeric ball.

### Detailed description of the invention

It will be immediately apparent that innumerable variations and modifications (for example relating to shape, dimensions, arrangements and parts with equivalent functionality) may be made to what has been described without departing from the scope of the invention as appears in the appended claims.

In fact, with the present invention, a new and/or alternative solution to the solutions known thus far is proposed and in particular it is proposed to remedy one or more of the drawbacks or problems referred to above, and/or to satisfy one or more requirements felt in the art, and in particular inferrable from the foregoing.

The various figures therefore show a magnetotherapy and compressive microvibration apparatus for the tissues comprising a handpiece 100 consisting of a head portion 200, a body 300 and a handle 400.

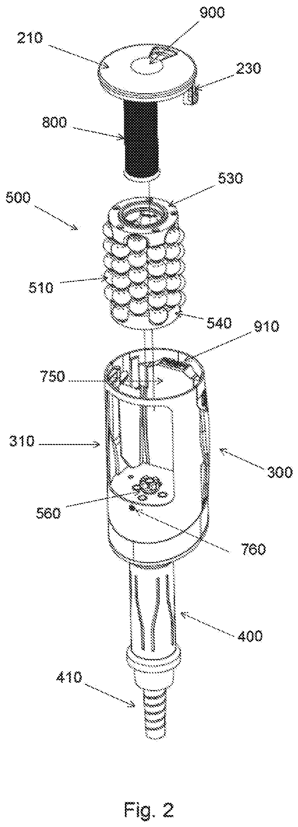

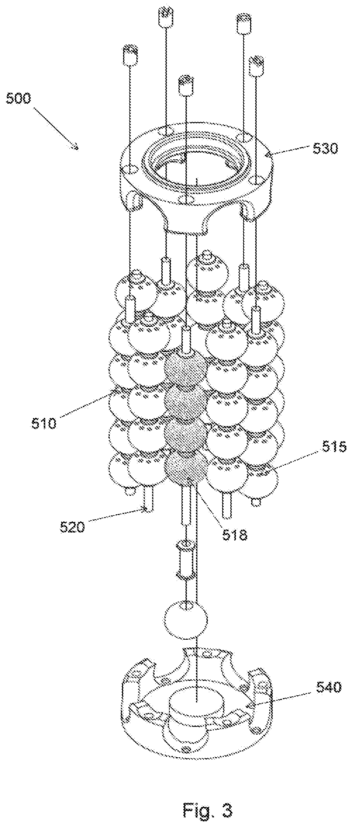

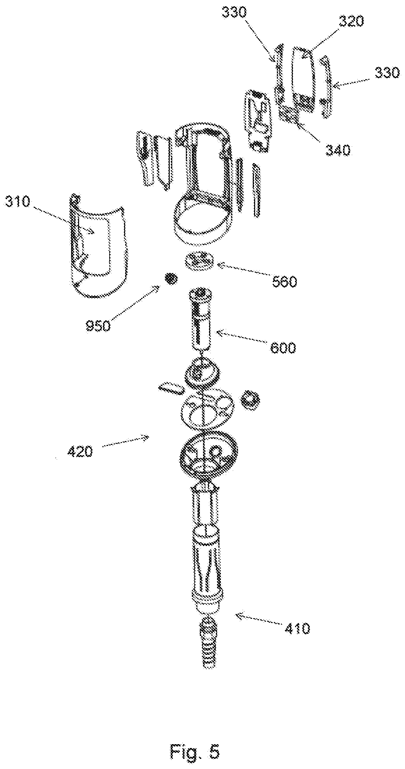

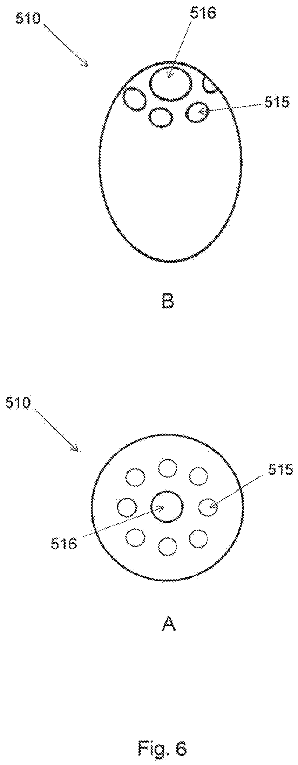

As specifically shown in Fig. 2, said handpiece 100 in the head portion 200 and in the body 300 has a recess adapted to house a rotor 500 adapted to carry out work in the radial direction with respect to the axis of the access hole made on said head portion 200. Said rotor 500, as shown in Fig. 3, is shaped like a hollow cylinder, which on the lateral surface has a plurality of balls 510 inserted through the longitudinal central channels 516 (see Fig. 6) on pins 520, so as to allow free rotation on themselves, in which each pin 520 is common to at least four balls 510 and in which the axis of rotation of the balls 510 and of the rotor 500 coincides with that of insertion into the recess of the handpiece 100 and in which said pins 520 are constrained respectively to a head ring nut 530 and to a ring nut of the handle 540. Said ring nut of the handle 540, shown specifically in Fig. 2, comprises a central fitting system adapted to be inserted on a rotation mechanism 560 forming part of an electric motor 600 (see Fig. 5) housed within said handle 400 and in which said electric motor 600 is connected via isolating support means 420 and clamping means 410 to a cable 700 connected in turn to a computer terminal (not shown).

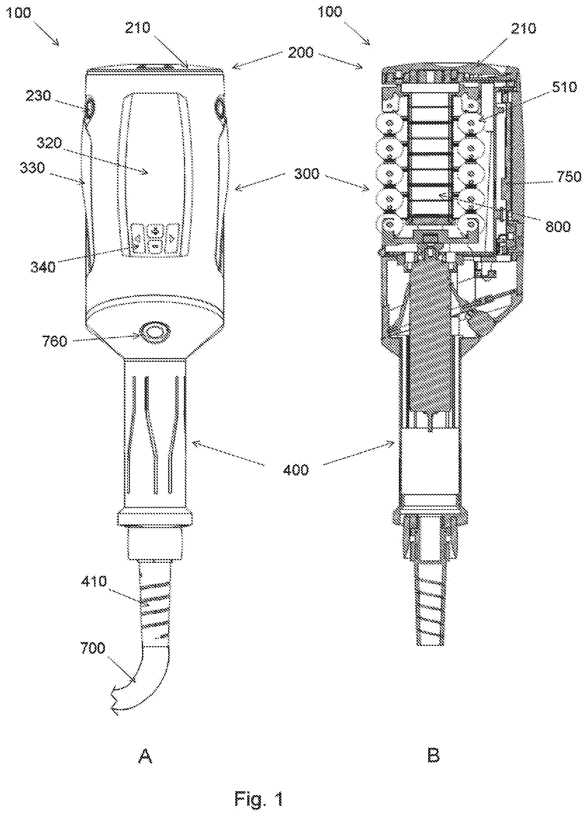

The rotation of the rotor 500 is such as to cause the passive rotation of each individual ball 510 on itself, every time it slides on the patient's tissue, through a rectangular opening 310 shown in Fig. 2 and made on said body 300 of the handpiece 100. As shown in Figs. 1A and 5, on the body 300 of the handle 100 in a position opposite to that of the rectangular opening 310, an interface is made comprising a display 320, a plurality of indicators 330 and a plurality of buttons 340.

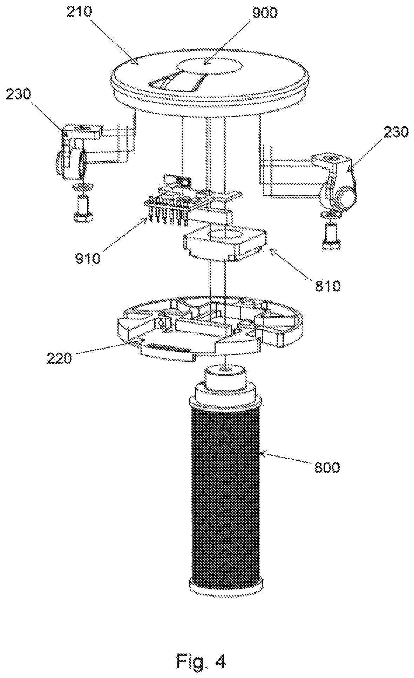

With reference to Fig. 4, the head portion 200 comprises a flange 220, in which one face of the same is adapted to allow the rotation of said head ring nut 530 (Fig. 2) while the other face is constrained to a plug 210 of the handpiece. Said cap 210 is removable and is connected to the head portion 200 of the handpiece 100 through at least a common pair of reversible connection systems 230 and therefore capable of being removed to allow easy extraction of the rotor 500 after each session to replace it or for related sanitization.

A specific feature of the present invention is that of comprising the balls 510 made of an elastomeric material having different degrees of elasticity and which, as shown in detail in Figs. 2, 3 and 6A-B, have a particular internal structure consisting of multiple longitudinal internal channels 515 made in parallel around said longitudinal central channel 516.

The association of said elastomeric property with the presence of said longitudinal internal channels 515 is adapted to give said balls 510 well-defined viscoelastic properties and exploited to absorb in a calibrated manner part of the energy transferred by the handpiece 100 on the patient's body area being treated. As shown in Fig. 3, another innovative feature derives from the fact that the creation of rows with balls 510 each having the same viscoelastic property associated with the presence in the rotor of at least a row of rigid balls 518 (see Fig. 3) without said longitudinal internal channels 515 and therefore capable of exerting a hypercompressive effect with respect to the analogous balls 510, gives the inventive device the ability to produce certain compressive harmonic waves amplified on the body area being treated, in addition to the classic compressive effect already existing in the art. Therefore, it is intuitive to consider that the frequency, duration and shape of said compressive harmonic waves also derive directly from the arrangement of said rows of balls 510 having viscoelastic properties associated with the presence of at least a row of rigid balls 518.

It forms a further specific feature of the present invention that of comprising in the cavity of said rotor 500 an inducer device 800, specifically shown in Figs. 1B, 2 and 4, consisting of at least a solenoid with a cylindrical shape, which engages a rotary bearing 810 through one end (Fig. 4) which in turn is connected to said head ring nut 530. The presence of said inducer device 800 inside the handpiece 100 is an aspect which particularly characterizes the present invention, since it allows obtaining a combined magnetotherapy treatment and a compressive microvibration treatment of the tissues.

Another specific feature of the present invention shown in Figs. 1A and 2 is that of making a pressure sensor 900 on the cap 210 of the handpiece adapted to detect the pressure of the rotor 500 exerted by the balls 510 on the tissue of the patient undergoing therapy. Also, said pressure sensor 900 results, through a connection device 910 consisting of a connector provided with a plurality of pins (Figs. 2 and 4), in connection with an electronic control unit 750 (Fig. 1).

A further specific feature of the present invention as shown in Fig. 5, is to provide said handpiece with a revolution sensor 950 located near the rotation mechanism 560 and adapted to detect the revolutions of the rotor in real time and therefore to send the data to said electronic control unit 750, which it is controlled by the computer terminal by means of a wiring system, which passes through said cable 700.

Specifically, a fundamental feature of the invention relates to the fact that said device inventively presents a mechanism for adjusting the intensity of the device according to the resistance encountered on the tissue and therefore relating to the presence of more or less incompressible fibrous areas and which represent an unequivocal sign of cellulite areas. In other words, the dedicated system of pressure 900 and revolution 950 sensors is adapted to ensure that, by placing the handpiece on the dermis only with its weight, the mechanical resistance encountered by the rotor balls while sliding on the patient's tissue is detected, thus detecting the type of normal, edematous or fibrous tissue with which the balls are in contact and therefore autonomously regulating the intensity of the device according to the detected resistance.

A particularly preferred embodiment intended to speed up the exchange between the numerous data in real time detected by said sensors of the handpiece 100 and the computer terminal consists of a wireless connection obtained via a wireless card housed in said electronic control unit 750 of the handpiece 100.

Said electronic control unit 750 is located close to and behind the interface and in addition manages the power supply to said inductor device 800 through said connection device 910.

Another innovative concept underlying the present invention provides for the presence at the area of the body 300 of the handpiece 100, specifically comprised between the handle 400 and the rectangular opening 310, of a temperature sensor 760 shown in Figs. 2 and 4, also connected to the electronic control unit 750, capable of detecting the temperature of the patient's integumentary apparatus and therefore of detecting areas of greater or lesser blood perfusion.

A further aspect, on the sidelines of all the technical solutions described up to now, but of particular relevance in the implementation of the invention, concerns the creation and development of a multiplicity of programs used in the data processing system and which create a software specially developed by the inventors, capable of being able to best and effectively perform all the functions deriving from the technical features described up to now.

For this purpose, said dedicated software is able to assist the operator in real time by showing through said display 320 and said indicators 330 both the pressure exerted in real time by the rotor 500 and therefore exerted by the balls 510 on the patient's tissue undergoing treatment and detected by said pressure sensor 900, and the number of revolutions of the rotor 500 detected in real time by the revolution sensor 950 and the tissue temperature of the patient detected by the temperature sensor 760.

Said dedicated software via the buttons 340 of the interface is adapted to allow the operator to be able to instantly vary all those parameters set at the origin of the session in the computer terminal on the basis of a standard basic setting or on the basis of the data present in the memory and obtained from the previous sessions of a specific patient. Specifically, the parameters set at the start of the session in the computer terminal are the speed of the rotor, the intensity of the impulses of the inductor and the feedback mechanism which links the automatic variation of the speed of the rotor on the basis of the temperature values detected on the body areas and the resistance that the balls encounter during contact with the patient's tissue.

Obviously what has just been described is optimized through the use of an application which allows prompt initial recognition achieved by attributing to the patient himself a unique recognition QR detected by a reader of the computer terminal. In fact, once the patient has been identified, said computer terminal will analyze the data stored, possibly also interfacing with a data center and therefore allowing for optimization of the treatment, as these surveys can be sent remotely to the various scientific or medical consultants, who can issue additional diagnoses or prescriptions to the patient himself.

The set of programs used in the data processing system and which implements the dedicated software includes a program capable of analyzing the data detected by the temperature sensor 760 during treatment and of drawing up on the computer terminal connected to said handpiece 100 a thermal map of the patient's body areas, which will be viewed by the operator before or during each treatment session, to understand both on which areas he/she will have to focus more on the basis of the pathology to be treated and to perform the initial setting of the software dedicated to the range of values he/she deems necessary.

It is therefore a specific object of the present invention to develop an effective medical apparatus including the relative dedicated software, capable of realizing the benefits deriving from a combined magnetotherapy treatment and a compressive microvibration treatment of the tissues in the treatment or alleviation of aesthetic disorders resulting from cellulite and all this through a non-invasive treatment and therefore suitable for all ages and not least without any contraindication.

A further and more general object is to be able to realize a combined magnetotherapy and compressive microvibration treatment of the tissues capable of being advantageously used in the improvement of disorders associated with vascular hemodynamics, the increase in interstitial fibrous tissue, lymphatic stasis and lymphedema, to painful states resulting from edematous conditions, all obtainable with a few applications.

Of course, the data provided herein are purely illustrative and absolutely non-limiting of the scope of the present invention, since they serve exclusively to enable a person skilled in the art to understand some possible applications and embodiments of the invention. Therefore, from the foregoing it is clear that the inventive concept is adaptable to the particular needs of each individual case and that therefore different modifications can be made to the above description without departing from its scope of protection as defined by the following dependent claims.

## Claims

1. Magnetotherapy and compressive microvibration apparatus for the tissues comprising a handpiece (100) constituted by a head portion (200), by a body (300) and by a handle (400), in which in the head portion (200) and in the body (300) there is a recess adapted to house a rotor (500) adapted to carry out work in the radial direction with respect to the axis of the access hole made on said head portion (200), in which said rotor (500) has the form of a hollow cylinder, having on the lateral surface a plurality of balls (510) that are free to rotate on themselves and inserted on pins (520) by means of the longitudinal central channels (516), in which each pin (520) is common to at least four balls (510) and in which the rotation axis of the balls (510) and of the rotor (500) coincide with that of insertion in the recess of the handpiece (100), in which said pins (520) are constrained respectively to a head ring nut (530) and to a ring nut of the handle (540), in which said ring nut of the handle (540) comprises a central fitting system adapted to be inserted on a rotation mechanism (560) constituting part of an electric motor (600) housed within said handle (400), in which said electric motor (600) is electrically connected, by means of isolating (420) and tightening (410) support means, to a cable (700) in turn connected to a computer terminal, in which the rotation of the rotor (500) is such to cause the passive rotation of each single ball (510) on itself each time that this comes into contact with the tissue of the patient, by means of a rectangular opening (310) made on said body (300), in which on the body (300), in a position opposite that of the rectangular opening (310), an interface is attained comprising a display (320), a plurality of indicators (330) and a plurality of buttons (340), in which the head portion (200) comprises a flange (220) having one face adapted to allow the rotation of said head ring nut (530) and having the other face constrained to a cap (210), in which said cap (210) is removable and is connected to the head portion (200) by means of at least a common pair of reversible connection systems (230) and **characterized in that** said balls (510) are made of elastomeric material having different grades of elasticity with an internal structure constituted by multiple longitudinal internal channels (515) attained parallel around said longitudinal central channel (516), **in that** the presence of said longitudinal internal channels (515) is adapted to confer to said balls (510) viscoelastic properties that are defined and exploited for controllably absorbing the pressure transferred by the handpiece (100) onto the tissue under treatment of the patient, **in that** in the cavity of said rotor (500) there is an inducer device (800) constituted by at least a solenoid with cylindrical shape constrained at one end to a rotary bearing (810), **in that** said rotary bearing (810) is constrained to said head ring nut (530), **in that** on said cap (210) there is a pressure sensor (900) adapted to detect the pressure of the rotor (500) exerted by the balls (510) on the tissue of the patient during the treatment, **in that** said pressure sensor (900) is connected with an electronic control unit (750) by means of a connection device (910) constituted by a connector provided with a plurality of pins, **in that** in proximity to the rotation mechanism (560) there is a revolution sensor (950) adapted to detect the revolution of the rotor (500) and to send the data to said electronic control unit (750), **in that** said electronic control unit (750) is controlled by the computer terminal both by means of a wiring system which passes by means of said cable (700) and by means of a wireless connection obtained by means of a wireless card, **in that** said electronic control unit (750) is situated in proximity to and behind said interface and manages, by means of said connection device (910), the power supply to said inducer device (800), **in that** at the area of the body (300) comprised between the handle (400) and the rectangular opening (310), there is a temperature sensor (760) connected to the electronic control unit (750) adapted to detect zones of greater or lesser perfusion of the tissue of the patient during the execution of the treatment.

2. Magnetotherapy and compressive microvibration apparatus for the tissues according to claim 1, **characterized in that** said balls (510) belonging to each row have the same viscoelastic property, **in that** in said rotor (500), there is at least a row of rigid balls (518) lacking said longitudinal internal channels (515) and adapted to carry out a hypercompressive effect that exploits the muscle as resistance, at the time when they cyclically enter into contact with the body tissue and **in that** the presence of the rigid balls (518) and of the balls (510) during the rotation of the rotor is adapted to produce compressive harmonic waves amplified over the body area under treatment, in addition to said compressive effect.

3. Magnetotherapy and compressive microvibration apparatus for the tissues according to any one of the claims **characterized in that** the presence of the pressure sensor (900) and of the revolution sensor (950) is adapted to detect the mechanical resistance encountered by the rigid balls (518) and by the balls (510) when they come into contact with the tissue of the patient and **in that** said resistance is adapted to indicate which type of tissue, normal, edematous or fibrous, is in contact with the aforesaid balls.

4. Magnetotherapy and compressive microvibration apparatus for the tissues according to any one of the preceding claims comprising a multiplicity of programs employed in the data processing system and attaining a dedicated and suitably processed software, in which said dedicated software is **characterized in that** by means of said display (320) and said indicators (330), the pressure is indicated which is exerted by the rotor (500) on the tissue of the patient subjected to therapy and detected by said pressure sensor (900) and **in that** by means of said display (320) and said indicators (330), the tissue temperature of the various tissue zones of the patient is indicated in real time and detected by the temperature sensor (760).

5. Magnetotherapy and compressive microvibration apparatus for the tissues according to any one of the preceding claims **characterized in that** said dedicated software is adapted to self-regulate in real time the speed of the rotor (500) and the intensity of the pulses of the inducer device (800) both based on the feedback mechanism determined by the values of the temperature of the body zones detected by said temperature sensor (760) and based on the values of the resistance that the balls encounter during the contact with the tissue of the patient and detected by said pressure sensor (900) and by the revolution sensor (950).

6. Magnetotherapy and compressive microvibration apparatus for the tissues according to any one of the preceding claims **characterized in that** said dedicated software is adapted to be configured by the operator at the start of the session by means of the computer terminal and **in that** said configuration occurs based on the data present in the memory and obtained from the preceding sessions of the patient under treatment and **in that** said dedicated software is adapted to be configured by the operator at the start of the session so as to set the value of the speed of the rotor (500) and the intensity of the pulses of the inducer device (800).

7. Magnetotherapy and compressive microvibration apparatus for the tissues according to any one of the preceding claims **characterized in that** said dedicated software is adapted to allow the operator by means of the buttons (340) of the interface of varying in real time the parameters set at the origin of the session in the computer terminal.

8. Magnetotherapy and compressive microvibration apparatus for the tissues according to any one of the preceding claims **characterized in that** said dedicated software is adapted for the recognition of the patient by means of the attribution of a unique recognition QR to the patient himself/herself and detected by a reader placed in the computer terminal, **in that** said computer terminal, once the identification is executed, analyzes the data in the memory, **in that** the data stored in real time is sent remotely to scientific experts or doctors.

9. Magnetotherapy and compressive microvibration apparatus for the tissues according to any one of the preceding claims **characterized in that** said dedicated software is adapted to analyze the data detected by the temperature sensor (760) during the treatment and **in that** on the computer terminal, a thermal map of the body zones of the patient is processed and graphically represented.

10. Magnetotherapy and compressive microvibration apparatus for the tissues according to any one of the preceding claims **characterized in that** said interface is constituted by at least a touchscreen suitable for the interaction in real time with the operator.

## Patentansprüche

1. Magnetotherapie- und kompressive Mikrovibrationsvorrichtung für Gewebe, die ein Handstück (100) aufweist, das durch einen Kopfteil (200), durch einen Körper (300) und durch einen Griff (400) gebildet ist, wobei in dem Kopfteil (200) und in dem Körper (300) eine Aussparung vorhanden ist, die dazu angepasst ist, einen Rotor (500) aufzunehmen, der dazu angepasst ist, eine Arbeit in der radialen Richtung in Bezug auf die Achse der in dem Kopfteil (200) ausgebildeten Zugangsöffnung auszuführen, wobei der Rotor (500) die Form eines Hohlzylinders aufweist, der an der lateralen Oberfläche eine Vielzahl von Kugeln (510) aufweist, die frei um sich selbst drehbar sind und mittels der länglichen zentralen Kanäle (516) auf Stifte (520) aufgesteckt sind, wobei jeder Stift (520) zumindest vier Kugeln (510) gemeinsam ist und wobei die Drehachse der Kugeln (510) und des Rotors (500) mit der des Einsetzens in die Aussparung des Handstücks (100) zusammenfallen, wobei die Stifte (520) jeweils an einer Kopfringmutter (530) und an einer Ringmutter des Griffs (540) eingeschränkt sind, wobei die Ringmutter des Griffs (540) ein zentrales Passsystem aufweist, das dazu angepasst ist, auf einen Drehmechanismus (560) aufgesetzt zu werden, der einen Teil eines Elektromotors (600) bildet, eingesetzt werden kann, der in dem Griff (400) untergebracht ist, wobei der Elektromotor (600) mittels isolierender (420) und festmachender (410) Halterungsmittel mit einem Kabel (700) elektrisch verbunden ist, das wiederum mit einem Computerterminal verbunden ist, wobei die Drehung des Rotors (500) derart ist, dass sie die passive Drehung jeder einzelnen Kugel (510) um sich selbst jedes Mal, wenn diese mit dem Gewebe des Patienten in Kontakt kommt, bewirkt, und zwar mittels einer an dem Körper (300) erzeugten rechteckigen Öffnung (310), wobei an dem Körper (300) in einer Position gegenüber der der rechteckigen Öffnung (310) eine Schnittstelle erreicht wird, die eine Anzeige (320), mehrere Anzeiger (330) und mehrere Tasten (340) aufweist, wobei der Kopfteil (200) einen Flansch (220) mit einer Seite aufweist, die dazu angepasst ist, die Drehung der Kopfringmutter (530) zu ermöglichen, und bei dem die andere Seite an einer Kappe (210) eingeschränkt ist, wobei die Kappe (210) abnehmbar ist und mittels zumindest eines gewöhnlichen Paares reversibler Verbindungssysteme (230) mit dem Kopfteil (200) verbunden ist, und **dadurch gekennzeichnet ist, dass** die Kugeln (510) aus elastomerem Material mit unterschiedlichen Elastizitätsgrade hergestellt sind, mit einer inneren Struktur, die durch mehrere längliche innere Kanäle (515) gebildet ist, die parallel um den länglichen zentralen Kanal (516) herum erreicht sind, dadurch, dass das Vorhandensein der länglichen innernen Kanäle (515) dazu angepasst ist, den Kugeln (510) viskoelastische Eigenschaften zu verleihen, die definiert sind und genutzt werden, um den durch das Handstück (100) auf das behandelte Gewebe des Patienten übertragenen Druck kontrollierbar zu absorbieren, dadurch, dass in dem Hohlraum des Rotors (500) eine Induziereinrichtung (800) vorhanden ist, die durch zumindest ein Solenoid mit zylindrischer Form gebildet ist, das an einem Ende an einem Drehlager (810) eingeschränkt ist, dadurch, dass das Drehlager (810) an der Kopfringmutter (530) eingeschränkt ist, dadurch, dass auf der Kappe (210) ein Drucksensor (900) vorhanden ist, der dazu angepasst ist, den Druck des Rotors (500) zu erfassen, der während der Behandlung durch die Kugeln (510) auf das Gewebe des Patienten ausgeübt wird, dadurch, dass der Drucksensor (900) mittels einer Verbindungseinrichtung (910), die durch einen mit einer Vielzahl von Stiften versehenen Verbinder gebildet ist, mit einer elektronischen Steuereinheit (750) verbunden ist, dadurch, dass in der Nähe des Drehmechanismus (560) ein Drehsensor (950) vorhanden ist, der dazu angepasst ist, die Drehung des Rotors (500) zu erfassen und die Daten an die elektronische Steuereinheit (750) zu senden, dadurch, dass die elektronische Steuereinheit (750) durch das Computerterminal sowohl mittels eines Verkabelungssystems, das über das Kabel (700) verläuft, als auch mittels einer drahtlosen Verbindung, die über eine drahtlose Karte hergestellt wird, gesteuert wird, dadurch, dass die elektronische Steuereinheit (750) in der Nähe und hinter der Schnittstelle angeordnet ist und mittels der Verbindungseinrichtung (910) die Leistungsversorgung der Induziereinrichtung (800) besorgt, dadurch, dass im Bereich des Körpers (300), der zwischen dem Griff (400) und der rechteckigen Öffnung (310) besteht, ein Temperatursensor (760) vorhanden ist, der mit der elektronischen Steuereinheit (750) verbunden ist und dazu angepasst ist, während der Durchführung der Behandlung Zonen mit größerer oder geringerer Durchblutung des Gewebes des Patienten zu erfassen.

2. Magnetotherapie- und kompressive Mikrovibrationsvorrichtung für Gewebe gemäß Anspruch 1, **dadurch gekennzeichnet, dass** die zu jeder Reihe gehörenden Kugeln (510) die gleiche viskoelastische Eigenschaft aufweisen, dadurch, dass in dem Rotor (500) zumindest eine Reihe starrer Kugeln (518) vorhanden ist, die keine länglichen inneren Kanäle (515) aufweisen und dazu angepasst sind, eine hyperkompressive Wirkung auszuüben, die den Muskel als Widerstand nutzt, wenn sie zyklisch mit dem Körpergewebe in Kontakt kommen, und dadurch, dass das Vorhandensein der starren Kugeln (518) und der Kugeln (510) während der Drehung des Rotors dazu angepasst ist, zusätzlich zu der kompressiven Wirkung kompressive harmonische Wellen zu erzeugen, die über den behandelten Körperbereich verstärkt werden.

3. Magnetotherapie- und kompressive Mikrovibrationsvorrichtung für Gewebe gemäß einem der Ansprüche, **dadurch gekennzeichnet, dass** das Vorhandensein des Drucksensors (900) und des Drehsensors (950) dazu angepasst ist, den mechanischen Widerstand zu erfassen, auf den die starren Kugeln (518) und die Kugeln (510) stoßen, wenn sie mit dem Gewebe des Patienten in Kontakt kommen, und dadurch, dass der Widerstand dazu angepasst ist, anzuzeigen, welche Art von Gewebe, normal, ödematös oder fibrös, mit den vorgenannten Kugeln in Kontakt steht.

4. Magnetotherapie- und kompressive Mikrovibrationsvorrichtung für Gewebe gemäß einem der vorangehenden Ansprüche, die eine Mehrzahl von Programmen aufweist, die in dem Datenverarbeitungssystem eingesetzt werden und eine dedizierte und geeignet verarbeitete Software ergeben, wobei die dedizierte Software **dadurch gekennzeichnet ist, dass** mittels der Anzeige (320) und der Anzeiger (330) der durch den Rotor (500) auf das der Therapie unterzogene Gewebe des Patienten ausgeübte und durch den Drucksensor (900) erfasste Druck angezeigt wird, und dadurch, dass mittels der Anzeige (320) und der Anzeiger (330) die Gewebetemperatur der verschiedenen Gewebezonen des Patienten in Echtzeit angezeigt und durch den Temperatursensor (760) erfasst wird.

5. Magnetotherapie- und kompressive Mikrovibrationsvorrichtung für Gewebe gemäß einem der vorangehenden Ansprüche, **dadurch gekennzeichnet, dass** die dedizierte Software dazu angepasst ist, die Geschwindigkeit des Rotors (500) und die Intensität der Impulse der Induziereinrichtung (800) in Echtzeit selbst zu regulieren, und zwar sowohl basierend auf dem Rückkopplungsmechanismus, der durch die Werte der durch den Temperatursensor (760) erfassten Temperaturen der Körperzonen bestimmt wird, und als auch basierend auf den Werten des Widerstands, auf den die Kugeln während des Kontakts mit dem Gewebe des Patienten stoßen und der durch den Drucksensor (900) und durch den Drehsensor (950) erfasst wird.

6. Magnetotherapie- und kompressive Mikrovibrationsvorrichtung für Gewebe gemäß einem der vorangehenden Ansprüche, **dadurch gekennzeichnet, dass** die dedizierte Software dazu angepasst ist, durch den Bediener zu Beginn der Sitzung mittels des Computerterminals konfiguriert zu werden, und dadurch, dass die Konfiguration basierend auf den Daten, die in dem Speicher vorhandenen sind und aus den vorangegangenen Sitzungen des behandelten Patienten gewonnen wurden, erfolgt, und dadurch, dass die dedizierte Software dazu angepasst ist, den Wert der Geschwindigkeit des Rotors (500) und die Intensität der Pulse der Induziereinrichtung (800) einzustellen.

7. Magnetotherapie- und kompressive Mikrovibrationsvorrichtung für Gewebe gemäß einem der vorangehenden Ansprüche, **dadurch gekennzeichnet, dass** die dedizierte Software dazu angepasst ist, es dem Bediener mittels der Tasten (340) der Schnittstelle zu ermöglichen, die am Anfang der Sitzung in dem Computerterminal eingestellten Parameter in Echtzeit zu ändern.

8. Magnetotherapie- und kompressive Mikrovibrationsvorrichtung für Gewebe gemäß einem der vorangehenden Ansprüche, **dadurch gekennzeichnet, dass** die dedizierte Software zur Erkennung des Patienten mittels der Zuweisung eines eindeutigen Erkennungs-QRs an den Patienten/die Patientin selbst, der durch einen in dem Computerterminal angeordneten Leser ermittelt wird, angepasst ist, dadurch, dass das Computerterminal, sobald die Erkennung ausgeführt wird, die Daten in dem Speicher analysiert, dadurch, dass die in Echtzeit gespeicherten Daten ferngesteuert an wissenschaftliche Experten oder Ärzte gesandt werden.

9. Magnetotherapie- und kompressive Mikrovibrationsvorrichtung für Gewebe gemäß einem der vorangehenden Ansprüche, **dadurch gekennzeichnet, dass** die dedizierte Software dazu angepasst ist, die durch den Temperatursensor (760) während der Behandlung erfassten Daten zu analysieren, und dadurch, dass auf dem Computerterminal eine Wärmekarte der Körperzonen des Patienten verarbeitet und grafisch dargestellt wird.

10. Magnetotherapie- und kompressive Mikrovibrationsvorrichtung für Gewebe gemäß einem der vorangehenden Ansprüche, **dadurch gekennzeichnet, dass** die Schnittstelle zumindest aus einem Touchscreen besteht, der für die Interaktion mit dem Bediener in Echtzeit geeignet ist.

## Revendications

1. Appareil de magnétothérapie et de microvibration compressive pour les tissus comprenant une pièce à main (100) composée d'une partie de tête (200), d'un corps (300) et d'une poignée (400), dans lequel est présent dans la partie de tête (200) et dans le corps (300) un évidement conçu pour loger un rotor (500) conçu pour effectuer un travail dans une direction radiale par rapport à un axe d'un trou d'accès réalisé sur ladite partie de tête (200), dans lequel ledit rotor (500) a la forme d'un cylindre creux, présentant sur la surface latérale une pluralité de billes (510) qui sont libres de tourner sur elles-mêmes et insérées sur des tiges (520) au moyen de canaux centraux longitudinaux (516), dans lequel chaque tige (520) est commune à au moins quatre billes (510) et dans lequel l'axe de rotation des billes (510) et du rotor (500) correspond à celui d'insertion dans l'évidement de la pièce à main (100), dans lequel lesdites tiges (520) sont contraintes respectivement sur un écrou à œil de tête (530) et sur un écrou à œil de la poignée (540), dans lequel ledit écrou à œil de la poignée (540) comprend un système de raccord central conçu pour être inséré sur un mécanisme de rotation (560) constituant une partie d'un moteur électrique (600) logé à l'intérieur de ladite poignée (400), dans lequel ledit moteur électrique (600) est connecté électriquement, à l'aide de moyens de support isolants (420) et de serrage (410), à un câble (700) connecté à son tour à un terminal informatique, dans lequel la rotation du rotor (500) est de nature à provoquer la rotation passive de chaque bille (510) sur elle-même à chaque fois que celle-ci entre en contact avec le tissu du patient, à l'aide d'une ouverture rectangulaire (310) réalisée sur ledit corps (300), dans lequel sur le corps (300), dans une position opposée à celle de l'ouverture rectangulaire (310), une interface est obtenue comprenant un dispositif d'affichage (320), une pluralité d'indicateurs (330) et une pluralité de boutons (340), dans lequel la partie de tête (200) comprend une bride (220) présentant une face conçue pour permettre la rotation dudit écrou à œil de tête (530) et présentant l'autre face contrainte sur un capuchon (210),
dans lequel ledit capuchon (210) est amovible et est relié à la partie de tête (200) au moyen d'au moins une paire commune de systèmes de connexion réversibles (230) et **caractérisé en ce que** lesdites billes (510) sont réalisées en matériau élastomère ayant différents degrés d'élasticité avec une structure interne composée de multiples canaux internes longitudinaux (515) réalisés parallèlement autour dudit canal central longitudinal (516), **en ce que** la présence desdits canaux internes longitudinaux (515) est conçue pour conférer auxdites billes (510) des propriétés viscoélastiques qui sont définies et exploitées pour absorber de manière régulable la pression transférée par la pièce à main (100) sur le tissu sous traitement du patient, **en ce que** dans la cavité dudit rotor (500) est présent un dispositif inducteur (800) composé d'au moins un solénoïde de forme cylindrique contraint sur une extrémité à un palier rotatif (810), **en ce que** ledit palier rotatif (810) est contraint audit écrou à œil de tête (530), **en ce que** sur ledit capuchon (210) est présent un capteur de pression (900) conçu pour détecter la pression du rotor (500) exercée par les billes (510) sur les tissus du patient pendant le traitement, **en ce que** ledit capteur de pression (900) est connecté à une unité de commande électronique (750) au moyen d'un dispositif de connexion (910) composé d'un connecteur muni d'une pluralité de tiges, **en ce qu'**à proximité du mécanisme de rotation (560) est présent un capteur de révolution (950) conçu pour détecter la révolution du rotor (500) et pour envoyer les données à ladite unité de commande électronique (750), **en ce que** ladite unité de commande électronique (750) est commandée par le terminal informatique à la fois au moyen d'un système de câblage qui passe au moyen dudit câble (700) et au moyen d'une connexion sans fil obtenue au moyen d'une carte sans fil, **en ce que** ladite unité de commande électronique (750) est située à proximité de ladite interface et derrière celle-ci et gère, au moyen dudit dispositif de connexion (910), l'alimentation électrique dudit dispositif inducteur (800), **en ce qu'**au niveau de la zone du corps (300) comprise entre la poignée (400) et l'ouverture rectangulaire (310), est présent un capteur de température (760) connecté à l'unité de commande électronique (750) conçu pour détecter des zones de perfusion plus ou moins importante du tissu du patient pendant l'exécution du traitement.

2. Appareil de magnétothérapie et de microvibration compressive pour les tissus selon la revendication 1, **caractérisé en ce que** lesdites billes (510) appartenant à chaque rangée présentent la même propriété viscoélastique, et en ce dans ledit rotor (500) est présente au moins une rangée de billes rigides (518) dépourvues desdits canaux internes longitudinaux (515) et conçues pour réaliser un effet hyper-compressif qui exploite le muscle comme résistance, au moment où elles entrent cycliquement en contact avec le tissu corporel et **en ce que** la présence des billes rigides (518) et des billes (510) pendant la rotation du rotor est conçue pour produire des ondes harmoniques de compression amplifées sur une zone du corps sous traitement, en plus dudit effet compressif.

3. Appareil de magnétothérapie et de microvibration compressive pour les tissus selon l'une des revendications, **caractérisé en ce que** la présence du capteur de pression (900) et du capteur de révolution (950) est conçue pour détecter la résistance mécanique rencontrée par les billes rigides (518) et par les billes (510) lorsqu'elles entrent en contact avec le tissu du patient et **en ce que** ladite résistance est conçue pour indiquer quel type de tissu, normal, œdémateux ou fibreux, est en contact avec les billes précitées.

4. Appareil de magnétothérapie et de microvibration compressive pour les tissus selon l'une des revendications précédentes, comprenant une multiplicité de programmes employés dans le système de traitement de données et disposant d'un logiciel dédié et adéquatement traité, dans lequel ledit logiciel dédié est **caractérisé en ce qu'**au moyen dudit dispositif d'affichage (320) et desdits indicateurs (330), est indiquée la pression exercée par le rotor (500) sur le tissu du patient soumis à la thérapie et détectée par ledit capteur de pression (900) et **en ce qu'**au moyen dudit dispositif d'affichage (320) et desdits indicateurs (330), la température tissulaire des différentes zones tissulaires du patient est indiquée en temps réel et détectée par le capteur de température (760).

5. Appareil de magnétothérapie et de microvibration compressive pour les tissus selon l'une des revendications précédentes, **caractérisé en ce que** ledit logiciel dédié est conçu pour autoréguler en temps réel la vitesse du rotor (500) et l'intensité des impulsions du dispositif inducteur (800) à la fois sur la base du mécanisme de rétroaction déterminé par les valeurs de température des zones corporelles détectées par ledit capteur de température (760) et sur la base des valeurs de résistance que rencontrent les billes lors du contact avec le tissu du patient et détectées par ledit capteur de pression (900) et par le capteur de révolution (950).

6. Appareil de magnétothérapie et de microvibration compressive pour les tissus selon l'une des revendications précédentes, **caractérisé en ce que** ledit logiciel dédié est conçu pour être configuré par l'opérateur au début de la séance à l'aide du terminal informatique et **en ce que** ladite configuration intervient sur la base des données présentes dans la mémoire et obtenues à partir des séances précédentes du patient traité et **en ce que** ledit logiciel dédié est conçu pour être configuré par l'opérateur au début de la séance de manière à régler la valeur de la vitesse du rotor (500) et l'intensité des impulsions du dispositif inducteur (800).

7. Appareil de magnétothérapie et de microvibration compressive pour les tissus selon l'une des revendications précédentes, **caractérisé en ce que** ledit logiciel dédié est conçu pour permettre à l'opérateur, à l'aide des boutons (340) de l'interface, de faire varier en temps réel les paramètres réglés au début de la séance dans le terminal informatique.

8. Appareil de magnétothérapie et de microvibration compressive pour les tissus selon l'une des revendications précédentes, **caractérisé en ce que** ledit logiciel dédié est conçu pour la reconnaissance du patient au moyen de l'attribution d'un QR de reconnaissance unique au patient lui-même et détecté par un lecteur placé dans le terminal informatique, dans lequel ledit terminal informatique, une fois l'identification exécutée, analyse les données en mémoire, **en ce que** les données stockées en temps réel sont envoyées à distance à des experts scientifiques ou à des médecins.

9. Appareil de magnétothérapie et de microvibration compressive pour les tissus selon l'une des revendications précédentes, **caractérisé en ce que** ledit logiciel dédié est conçu pour analyser les données détectées par le capteur de température (760) pendant le traitement et **en ce que** sur le terminal informatique, une carte thermique des zones corporelles du patient est traitée et représentée graphiquement.

10. Appareil de magnétothérapie et de microvibration compressive pour les tissus selon l'une des revendications précédentes, **caractérisé en ce que** ladite interface est composée d'au moins un écran tactile approprié pour l'interaction en temps réel avec l'opérateur.
